# EUROPEAN PATENT APPLICATION

(11) **EP 3 207 866 A1**
(43) Date of publication of application: **23.08.2017**
(21) Application number: 16156398.6
(22) Date of filing: 18.02.2016
(51) Int. Cl.: A61B 5/04, A61B 5/00

(54) **VECTOR MAGNETOCARDIOGRAPHY METHOD AND VECTOR MAGNETOCARDIOGRAPHIC SYSTEM**

(71) Applicant: Biomagnetik Park GmbH, 21079 Hmburg (DE)
(72) Inventor: KIM, Byeongsoo, 22455 Hamburg (DE); EHRLEN, Malte, 20257 Hamburg (DE)
(74) Representative: Stüven, Ralf

(57) **Abstract**

The invention relates to a vector magnetocardiography method and a vector magnetocardiographic system carrying out the method. It is an object of the invention to provide an improved magnetocardiographic method, in particular in view of diagnosing ischemic heart disease. The method of the invention involves the calculation of the direction of an equivalent single current source ESCS or equivalent single magnetic source ESMS in relation to a reference direction which is independent of patient or heart position in order to compute a vector magnetocardiogram.

## Description

The invention relates to a vector magnetocardiography method and a vector magnetocardiographic system for carrying out the method.

Magnetocardiography (MCG) is a non-invasive method of recording magnetic fields generated by the electric activity of the heart, and can e.g. be used for clinical diagnosis of heart diseases, for example ischemic heart disease (IHD; see e.g. [2], [8], [10], [13], [14]).

Although frequently used nowadays, Magnetocardiography still has considerable drawbacks, especially in view of diagnosing IHD. The parameters used for diagnosis, including, for example, the field gradient [6], Magnetic map angle [12] and evaluations of different segments of the butterfly plot [7], have traditionally been represented in the sensor space. One major drawback of evaluating all parameters in the sensor space is system dependency, i.e. a direct comparison between different MCG devices is not possible. Furthermore, every time a system is modified, diagnostic parameters must be updated by conducting additional clinical studies.

Another drawback with known methods is that, while statistically significant for detection of ischemia, the variance is still too high for clinical application. A further problem with the prior diagnostic methods is that the field gradient is not well defined in the presence of monopole-like magnetic fields. These fields are associated with myocardial ischemia and as such, a diagnostic method targeting ischemia must be robust under these circumstances. There have been attempts to diagnose ischemia in the source space using pseudocurrent mapping [9]. It is possible to calculate the pseudocurrent map in the source space by minimum norm estimation [11]. The source space map, however, has similar drawbacks than the sensor space map, and additional errors from the pseudoinverse solution are introduced.

Vector Magnetocardiography (VMCG) is a magnetocardiographic method wherein all three orthogonal components of the magnetic heart vector are measured at the same location (see e.g. [10]). VMCG can be used to reconstruct a change of direction and strength of the magnetic heart vector over time, e.g. during the heart cycle.

It is an object of the invention to provide an improved magnetocardiographic method, in particular in view of diagnosing ischemic heart disease. In particular, it is an object of the invention to provide a magnetocardiographic method having no or minimal system dependency and variance.

In one aspect the problem is solved by a vector magnetocardiography method, the method comprising the steps of:
a. Measuring the amplitude and direction of the three orthogonal components of the magnetic field(s) generated by the heart of a subject during a period of heart activity using one or more magnetic field sensors,
b. Locating, using the data measured in step a, a reference source position for heart magnetic and/or electric activity, the reference source position being a point source, not necessarily inside the heart volume, suitable for representing the source of the magnetic and/or electric activity during said period of heart activity,
c. Evaluating a reference direction from the data measured in step a, the reference direction being the mean direction of the magnetic moment and/or electric current in the heart muscle volume during a period of magnetic and/or electric heart activity with known direction of the magnetic moment and/or electric current in relation to the heart anatomy,
d. Calculating, from the data measured in step a, an equivalent single current source ESCS or an equivalent single magnetic source ESMS at the reference source position located in step b, the ESCS or ESMS representing an electric heart vector EHV or a magnetic heart vector MHV, in relation to the reference direction evaluated in step c, and
e. Registering the EHV and/or MHV, calculated in step d during at least part of the heart activity in a vector magnetocardiogram.

By taking into account the position of the heart and using a suitable inverse solution method, e.g. multipole expansion in the source space, the invention solves the problems of system dependence, parameter variance and instability of the field gradient. The method of the invention is particulary suitable for providing information for diagnosing cardiac ischemia.

The inventors have found that the main reason for the failure of pseudocurrent mapping in the source space is that an attempt is made to solve the inverse problem using insufficient constraints on the sources. A normal pseudocurrent map consists of e.g. a grid of 16 by 16 (or even larger) infinitesimal current sources. Each current source has two degrees of freedom (amplitude in x- and y-direction). This means that in total, a solution must be found for 512 or more parameters. With the e.g. 64 channels of currently used MCG systems the maximum number of eigenvalues for the pseudoinverse of the leadfield matrix is at best 64, provided the channels are completely independent, which is not the case, because some eigenvalues must be cut to account for system and background noise. This number, however, is not enough to fit 512 parameters and find a unique solution to the inverse problem.

Instead of a map, the invention uses a single point source, e.g. a single magnetic multipole source, preferably a single dipole source, such that the number of degrees of freedom becomes managable (in the case of a dipole source, 3 positional and 3 directional degrees of freedom), and fewer independent eigenvalues to find a unique solution to the inverse problem are required. Although it is preferred to use e.g. a single magnetic dipole source, it is also possible to use another single source, e.g. a single infinitesimal current source. The inherent problem to this method, i.e. the strong dependency of the resulting solution from the position of this single source, is accounted for by the invention in that it provides a robust method to identify the optimal position.

In this context, it should be noted that this single point source does not represent any real physical activity of the heart, but rather it is an equivalent single magnetic or electric source (ESMS, ESCS), an approximation of volume electric activity by point magnetic or electric activity. The point source is deemed to represent the source for the magnetic and/or electric activity of the heart, and must therefore not necessarily lie inside the heart volume. Although most likely lying inside the heart volume, it may lie in the vicinity of the heart, i.e. outside the heart, e.g. below (posterior, i.e. lower on the z-axis) the real heart.

The invention involves finding the best fitting ESMS or ESCS through inverse solution of the magnetic field equations, preferably taking data measured during a suitable electric and/or magnetic heart activity. In order to find the best position for the point source, i.e. the ESMS or ESCS, a classical pseudocurrent map in the source space may, for example, be used. A magnetic field map can, for example, also be used. The skilled person is aware of known methods for the computation of such a pseudocurrent or magnetic field map. In general, any time interval with electric and/or magnetic heart activity can be taken for locating the point source. However, it is preferred to take a time interval with a strong electrical and/or magnetical activity. Further preferred, for finding the optimal location of the point source a time interval is used showing the strongest electric/magnetic activity during a period of strong electric/magnetic activity in the heart, such as during the R peak or during the T peak, e.g. at T_{max.} The position in this map of e.g. the strongest current is taken to determine the optimal location for the point source. It is to be noted here that, since any magnetic activity reflects an underlying electric activity, the position of any ESMS corresponds to the position of an equivalent single current source (ESCS). Therefore, for the method of the invention an ESCS can be used instead or even in addition to the ESMS. Further, it should be understood that, although measured signals originating from an electrically/magnetically active region of the heart are used for finding the optimal location of the point source, the point source itself must not necessarily represent a point within this region or even within the heart.

When the optimal position has been found, the inverse solution can be computed for each time point during the period of heart activity using an ESMS (or ESCS). Since the ESMS represents a magnetic heart vector (MHV), registering the change of this vector in regard to strength (amplitude) and direction over time is a way of representing an MCG measurement, and the term vector magnetocardiography (vector MCG) or VMCG is used for this method.

The invention thus provides a robust and stable method of registering the direction and amplitude of heart magnetic activity where the field gradient method fails.

Since current MCG systems measure the entire heart beat (sinus rythm), one possible way of representing the VMCG is as a trace of measurement intervals or segments, where each segment of the trace points from the previous magnetic moment vector to the next. The amplitude of the vector can, e.g. be represented by the length of the vector. It is then possible to observe angle and amplitude variations over the entire heart beat in a single diagram.

The invention also accounts for the large variance in current angle even in healthy subjects. Two factors contribute to the large variance in angle. First, patient position on the bed underneath the Dewar varies from measurement to measurement. Second, the position of the heart inside the torso varies from subject to subject. While strict procedures can account for the first factor and an imaging method such as CT scan or MRI could account for the second, this would represent a very arduous process and take away one of the advantages of MCG, i.e. that it is fast and independent of other diagnostic methods. The invention provides a single solution for solving the problems arising from the above factors.

A direction can be given by an angle from some reference direction. In prior art methods the positive x-axis is normally used as a reference, with the angle 0° in the xy plane, i.e. the frontal plane. According to the invention, however, the reference direction is defined in relation to the heart anatomy only, e.g. defined as always pointing to the apex of the heart, and therefore independent of patient or heart position. As a consequence, the measured angle would be invariant to patient and heart position.

From literature (see e.g. [10]), it is known that the direction of the electric current of the heart during the R peak is towards the apex of the ventricles. Further, during the R peak, the depolarization current is so strong that any ischemic injury current has little effect on the main current vector of the VMCG. In a preferred embodiment of the method of the invention, the VMCG direction during the R peak is therefore chosen as reference direction and set to 0°. The VMCG as recorded with the method of the invention is then unaffected by the drawbacks of pseudocurrent mapping and independent of external factors such as patient and heart position relative to the MCG device.

The term "magnetic field sensor" as used herein means a sensor being able to measure magnetic fields. According to the invention, one or more magnetic field sensors are used to measure both direction and magnitude (amplitude) of a magnetic field. This can e.g. be done by a single 3-axis magnetic field sensor, a plurality (an array) of 3-axis magnetic field sensors, or a plurality (an array) of 1-, 2-, and/or 3-axis magnetic field sensors. A "3-axis magnetic field sensor" is a magnetic field sensor measuring the components of the magnetic field in all three dimensions. The term encompasses sensors being composed of at least three magnetometers or gradiometers measuring the orthogonal x-, y-, and z-components of a magnetic field. The terms "1-axis magnetic field sensor" or "2-axis magnetic field sensor" refer to magnetic field sensors measuring only one or two of the three magnetic field components. An array of e.g. 1-axis magnetic field sensors oriented in such a manner that they measure orthogonal components of the magnetic field can e.g. also be used to measure both magnitude and direction of a magnetic field. SQUIDs ("superconducting quantum interference devices") are preferred as sensors.

As used herein, unless not stated otherwise or circumstances do not indicate otherwise, a reference to an x-axis in relation to the heart or the body of a human being corresponds to a reference to a right-to-left axis, a reference to an y-axis corresponds to a reference to a head-to-foot axis, and a reference to the z-axis corresponds to a reference to a anteroposterior axis.

The term "period of heart activity" relates to any period with an activity of the heart involving an electric current and/or the generation of a magnetic field by heart tissue. In particular, the term relates to the so-called sinus rhythm, i.e. the normal rhythm of the heart involving depolarization and repolarization of the atria and ventricles. In the sinus rhythm, depolarisation of the cardiac muscle begins at the sinoatrial node (also called sinus node or SA node) situated in the upper part of the wall of the right atrium, is conducted through the atrioventricular node (AV node) between the atria and ventricles to the bundle of His, the bundle branches and the Purkinje fibres. Repolarisation of the ventricles ends the cycle. Depolarisation and repolarisation of the atria and ventricles are reflected by three typical waves or wave complexes on a typical electrocardiogram (ECG), P wave, QRS complex and T wave. In the ECG, the P wave is usually considered to represent atrial depolarization, the QRS complex the depolarization of the right and left ventricles, and the T wave the repolarization of the ventricles.

The term "suitable magnetic and/or electric activity" refers to any magnetic and/or electric activity of the heart being reliably measurable. In particular, the term refers to a sufficiently strong magnetic and/or electric activity of the heart. Non-exhaustive examples for a suitable magnetic and/or electric activity are the heart activities during a period of heart activity represented by an R peak or T peak on an electrocardiogram (ECG).

The term "mean direction of the magnetic moment and/or electric current" refers to the average direction of the magnetic moment and/or electric current, considering the fact that the heart is a volume conductor.

The term "reference source position" refers to a point taken as the source of all electric and/or magnetic activity of the heart. The reference source position is thus a virtual point electric and/or magnetic source with parameters such that the field generated by this reference source is equivalent to that field which is measured from the heart volume. It should be noted that the reference source position, being a calculated point, may lie within the real volume source, i.e. within the heart, but can also lie outside the heart volume in the vicinity of the heart.

The term "known direction of the magnetic moment and/or electric current in relation to the heart anatomy" means the direction of the magnetic field and/or electric current in relation to a physical part of the heart, e.g. the tip of the heart, i.e. the apex of the left ventricle (Apex cordis).

The term "Registering the EHV and/or MHV during at least part of the heart activity" means registering the EHV and//or MHV, i.e. the magnitude (amplitude) and direction of the EHV/MHV, over time during a period of heart activity, e.g. during the sinus rhythm, or part thereof. The registered data may then be used to construct a magnetogram.

The term "inverse solution" means a solution to the inverse problem. The skilled person is familiar with this problem, and with methods to find an inverse solution, i.e. methods to solve an inverse problem. In the context of the invention the term "inverse solution" refers to methods for reconstructing the heart activity (i.e. the real electric and/or magnetic activity in the "source space", the source being the heart) with data measured in the "sensor space", i.e. outside the heart.

The term "subject" as used herein refers preferably to a vertebrate, further preferred to a mammal, and most preferred to a human.

The steps b to e of the method of the invention are preferably implemented by a software algorithm running on e.g. a computer.

In a preferred embodiment of the vector magnetocardiographic method of the invention, in step b mentioned above, for locating a reference source position, data measured during a strong magnetic and/or electric heart activity, preferably a during the strongest magnetic and/or electric activity of a period of strong heart activity are used for e.g. computing a pseudocurrent map and/or a magnetic field map. The procedures for computing a pseudocurrent map or a magnetic field map are known to the skilled person. A pseudocurrent map can, e.g. be computed by using a 16 x 16 grid, as known from the prior art. For finding the reference source position it is especially preferred to use the time interval having the strongest magnetic and/or electric activity during the R peak or T peak (e.g. at Tₘₐₓ), preferably during the R peak. The term "during the R peak or T peak" relates to a period of heart activity normally represented as R peak or T peak on an ECG.

Further preferred, in step c, the reference direction of the magnetic field and/or electric current in the heart is evaluated during the R peak. Since the direction of the electric current of the heart during the R peak is known to point towards the apex of the ventricles, and the R peak is also a period of strong heart activity generating a strong measurable signal, it is preferred to use the R peak in order to define the reference direction. However, any other period of magnetic and/or electric heart activity for which the direction of the magnetic field and/or electric current in relation to the heart anatomy is known or can be determined, are also suitable for establishing the reference direction.

In the vector magnetocardiographic method of the invention, it is especially preferred that for both the determination of the reference source position and the evaluation of the reference direction the period of heart activity corresponding to the R peak is used. The invention is, however, not restricted to using the R peak for both purposes.

In a further preferred embodiment of the vector magnetocardiographic method of the invention, the difference in direction and/or magnitude between the EHV and/or MHV at different points of the vector magnetocardiogram are calculated, the different points representing different points of time during the heart sinus rhythm. This is especially useful for diagnostic purposes. It has e.g. been found that the difference in direction and/or magnitude between the EHV and/or MHV at Tₘₐₓ and T_{end} is indicative of cardiac ischemia.

In a further aspect the invention relates to a vector magnetocardiographic system (VMCG system) being adapted for carrying out the method according to first aspect of the invention. The vector magnetocardiographic system of the invention preferably comprises
a. one or more magnetic field sensors for measuring direction and magnitude of the three orthogonal components of the magnetic field(s) generated by the heart of a subject during a period of heart activity,
b. a means for (automatically) locating, using the data measured by the one or more magnetic field sensors, a reference source position for heart magnetic and/or electric activity, the reference source position being a point source suitable for representing the source of the magnetic and/or electric activity during said magnetic and/or electric heart activity, preferably using data during the strongest magnetic and/or electric heart activity during a period of strong magnetic and/or electric heart activity,
c. a means for (automatically) evaluating a reference direction from the data measured by the one or more magnetic field sensors, the reference direction being the mean direction of the magnetic moment and/or electric current in the heart muscle volume during a period of magnetic and/or electric heart activity with known direction of the magnetic moment and/or electric current in relation to the heart anatomy,
d. a means for (automatically) calculating, from the data measured by the one or more magnetic field sensors, an equivalent single current source ESCS or an equivalent single magnetic source ESMS at the reference source position, the ESCS or ESMS representing an electric heart vector EHV or a magnetic heart vector MHV, in relation to the reference direction, and
e. a means for registering the EHV and/or MHV during at least part of the heart activity in a vector magnetocardiogram.

The VMCG system may be a VMCG system, the electronic data processing components of which have been especially adapted to carrying out the method of the first aspect of the invention, e.g. by hard-wiring an algorithm carrying out steps b to e of the inventive method. The VMCG system, however, can also be a standard VMCG system comprising a computer, e.g. a personal computer, running a computer program implementing an algorithm carrying out steps b to e of the inventive method.

In the following, the invention is described in more detail by way of an example and the attached figures for illustration purposes only.
Fig. 1. Schematic illustration for locating a reference source position using a pseudocurrent map.
Fig. 2. Exemplary VMCG of a healthy subject established using the method of the invention.
Fig. 3. 2-D representation of the VMCG of Fig. 2 corresponding to an equivalent single current source (ESCS). The current units given are [A] · 4π µ₀10⁻⁶, where µ₀ is the magnetic permeability of vacuum.
Fig. 4. 2-D representation of a VMCG of a patient with single vessel disease in the LAD. Current units of the axis are the same as in Fig. 3.

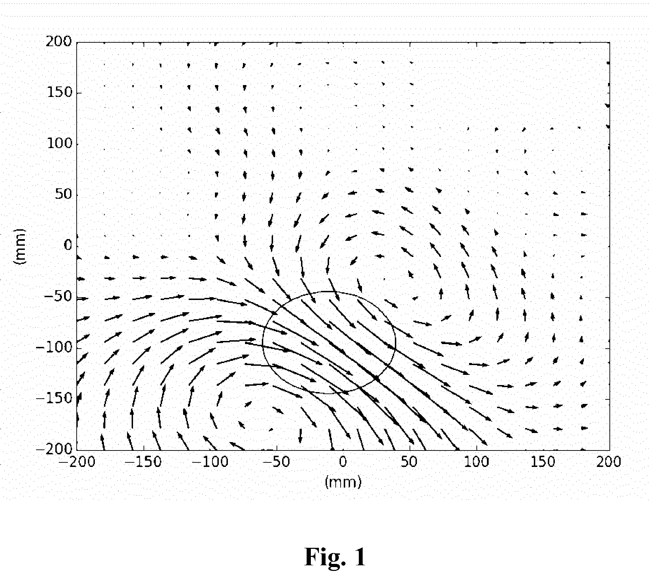

Figure 1 shows a standard pseudocurrent map for a 20 x 20 source grid, using 13 eigenvalues, for finding the optimal location of an ESMS. The pseudocurrent map was established from data measured during the R peak of the sinus rythm. The encircled area denotes the region of strongest electric activity during the R peak.

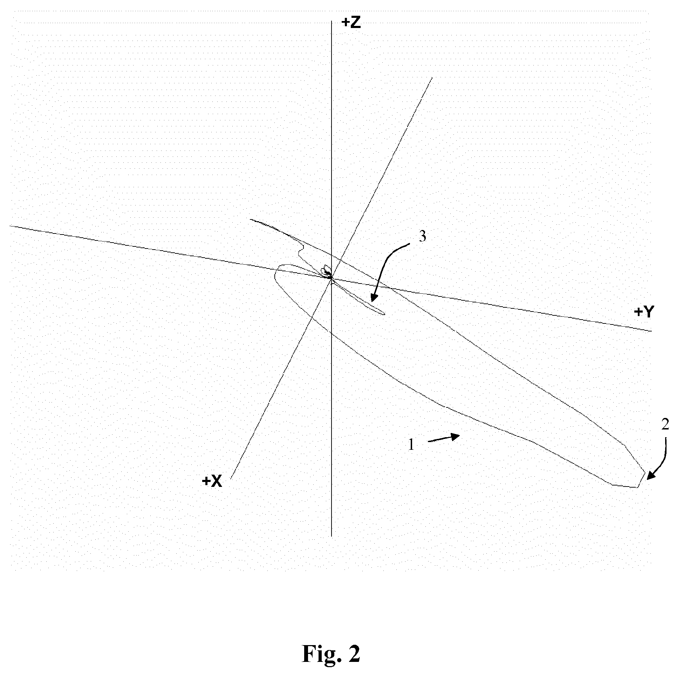

A VMCG of a healthy subject is depicted in Figure 2. The large loop denoted with the reference numeral 1 represents the QRS complex, and includes the R peak 2, the smaller loop 3 represents the T peak.

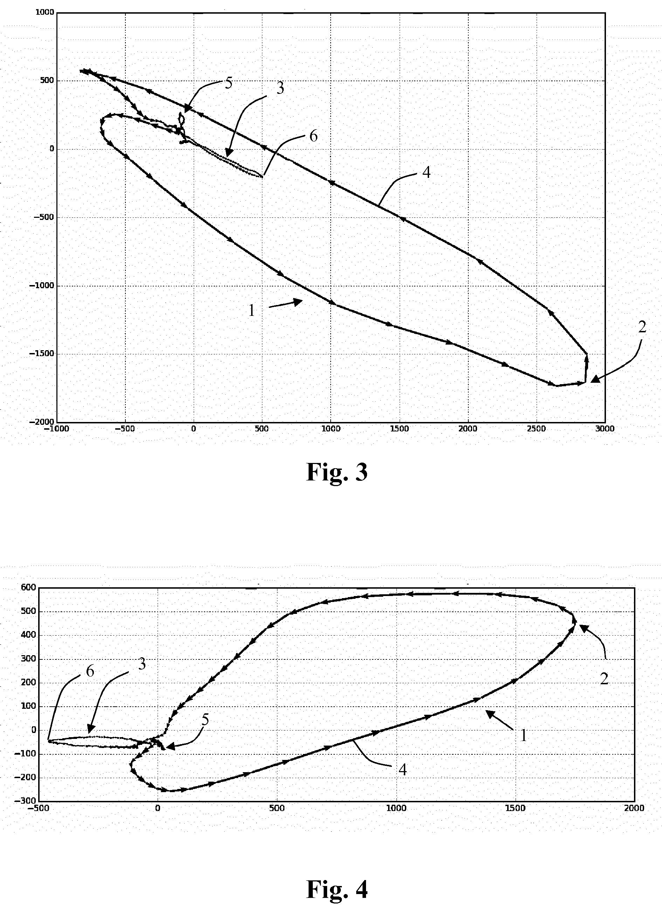

Figure 3 shows a 2-D representation of the of the VMCG of Fig. 2 corresponding to an equivalent single current source (ESCS). Such a representation is especially useful to facilitate angle measurements, i.e. a change in direction of MHV/EHV. The Q, R, S, T and even P waves are visible. The P wave at 125 ms is denoted with numeral 5, the R peak at 200 ms with numeral 2, Tₘₐₓ at 339 ms with numeral 6. The current directions during the entire sinus rythm are congruent with literature. Each segment 4 of the VMCG corresponds to an MHV for identical time intervals during the sinus rhythm, the length of each segment representing the amplitude of the magnetic field, and the orientation the direction in relation to the reference direction.

Fig. 4 is a 2-D representation of a VMCG of a subject with single vessel disease in the left anterior descending artery (LAD), confirmed by angiography. The current direction during the T-wave (Tₘₐₓ 6 at 381 ms) is about 180° off compared to the R peak (at 200 ms).

### References

[1] George E Burch. The history of vectorcardiography. Medical History, 29(S5):103-131, 1985.
[2] David Cohen, JC Norman, F Molokhia, and W Hood. Magnetocardiography of direct currents: St segment and baseline shifts during experimental myocardial infarction. Science, 172(3990):1329-1333, 1971.
[3] Harold W Draper, Catherine J Peffer, Friedman W Stallman, David Litmann, and Hubert V Pipberger. The corrected orthogonal electrocardiogram and vectorcardiogram in 510 normal men (frank lead system). Circulation, 30(6):853-864, 1964.
[4] Ernest Frank. An accurate, clinically practical system for spatial vectorcardiography. Circulation, 13(5):737-749, 1956.
[5] Wolfgang Haberkorn, Uwe Steinhoff, Martin Burghoff, Olaf Kosch, Andreas Morguet, and Hans Koch. Pseudocurrent density maps of electrophysiological heart, nerve or brain function and their physical basis. Biomagnetic Research and Technology, 4, 2006.
[6] Helena Hänninen et al., Multichannel magnetocardiography and body surface potential mapping in exercise-induced myocardial ischemia. University of Helsinki, 2002.
[7] Helena Hänninen, Panu Takala, Petri Korhonen, Lasse Oikarinen, Markku Mäkijärvi, Jukka Nenonen, Toivo Katila, and Lauri Toivonen. Features of st segment and t-wave in exercise-induced myocardial ischemia evaluated with multichannel magnetocardiography. Annals of medicine, 34(2):120-129, 2002.
[8] Helena Hänninen, Panu Takala, Markku Mäkijärvi, Juha Montonen, Petri Korhonen, Lasse Oikarinen, Jukka Nenonen, Toivo Katila, and Lauri Toivonen. Detection of exercise-induced myocardial ischemia by multichannel magnetocardiography in single vessel coronary artery disease. Annals of noninvasive electrocardiology, 5(2):147-157, 2000.
[9] Hyun Kyoon Lim, Namsik Chung, Kiwoong Kim, Young-Guk Ko, Hyukchan Kwon, Yong-Ho Lee, Jin-Bae Kim, Jung Rae Cho, Jin-Mok Kim, In-Seon Kim, et al. Reproducibility of quantitative estimate of magnetocardiographic ventricular depolarization and repolarization parameters in healthy subjects and patients with coronary artery disease. Annals of biomedical engineering, 35(1):59-68, 2007.
[10] Jaakko Malmivuo and Robert Plonsey. Bioelectromagnetism: principles and applications of bioelectric and biomagnetic fields. Oxford university press, 1995.
[11] JT Nenonen, MS Hämäläinen, and RJ Iimoniemi. Minimum-norm estimation in a boundary-element torso model. Medical and Biological Engineering and Computing, 32(1):43-48, 1994.
[12] JW Park and F Jung. Qualitative and quantitative description of myocardial ischemia by means of magnetocardiography. Biomedizinische Technik. Biomedical engineering, 49(10):267-273, 2004.
[13] Kirsten Tolstrup, Bo E Madsen, Jose A Ruiz, Stephen D Greenwood, Judeen Camacho, Robert J Siegel, H Caroline Gertzen, J-W Park, and Peter A Smars. Non-invasive resting magnetocardiographic imaging for the rapid detection of ischemia in subjects presenting with chest pain. Cardiology, 106(4):270-276, 2006.
[14] Satsuki YAMADA and Iwao YAMAGUCHI. Magnetocardiograms in clinical medicine: unique information on cardiac ischemia, arrhythmias, and fetal diagnosis. internal Medicine, 44(1):1-19, 2005.

## Claims

1. A vector magnetocardiography method, the method comprising the steps of:
a. Measuring direction and magnitude of the three orthogonal components of the magnetic field(s) generated by the heart of a subject during a period of heart activity using one or more magnetic field sensors,
b. Locating, using the data measured in step a, a reference source position for heart magnetic and/or electric activity, the reference source position being a point source suitable for representing the source of the magnetic and/or electric activity during said period of heart activity,
c. Evaluating a reference direction from the data measured in step a, the reference direction being the mean direction of the magnetic moment and/or electric current in the heart muscle volume during a period of magnetic and/or electric heart activity with known direction of the magnetic moment and/or electric current in relation to the heart anatomy,
d. Calculating, from the data measured in step a, an equivalent single current source ESCS or an equivalent single magnetic source ESMS at the reference source position located in step b, the ESCS or ESMS representing an electric heart vector EHV or a magnetic heart vector MHV, in relation to the reference direction evaluated in step c, and
e. Registering the EHV and/or MHV, calculated in step d, during at least part of the heart activity in a vector magnetocardiogram.

2. The vector magnetocardiographic method according to claim 1, wherein
a. in step b data measured during a strong magnetic and/or electric heart activity, preferably during the strongest magnetic and/or electric activity of a period of strong magnetic and/or electric heart activity, preferably during the R peak or T peak, is used for locating the reference source position, and/or
b. in step c the reference direction of the magnetic field and/or electric current in the heart is evaluated during the R peak.

3. The vector magnetocardiographic method according to claim 1 or 2, wherein, in step b, the reference source position is located by computing a pseudocurrent map and/or a magnetic field map using the data measured in step a.

4. The vector magnetocardiographic method according to any of the preceeding claims, wherein the difference in direction and/or magnitude between the EHV and/or MHV at different points of the vector magnetocardiogram are calculated, the different points representing different points of time during the heart sinus rhythm.

5. The vector magnetocardiographic method according to claim 4, wherein the difference in direction and/or amplitude between the EHV and/or MHV at Tₘₐₓ and T_{end} is calculated.

6. A vector magnetocardiographic system being adapted for carrying out the method according to one of claims 1 to 5.

7. A vector magnetocardiographic system according to claim 6, comprising
a. one or more magnetic field sensors for measuring direction and magnitude of the three orthogonal components of the magnetic field(s) generated by the heart of a subject during a period of heart activity,
b. a means for locating, using the data measured by the one or more magnetic field sensors, a reference source position for heart magnetic and/or electric activity, the reference source position being a point source suitable for representing the source of the magnetic and/or electric activity during said period of heart activity,
c. a means for evaluating a reference direction from the data measured by the one or more magnetic field sensors, the reference direction being the mean direction of the magnetic moment and/or electric current in the heart muscle volume during a period of magnetic and/or electric heart activity with known direction of the magnetic moment and/or electric current in relation to the heart anatomy,
d. a means for calculating, from the data measured by one or more magnetic field sensors, an equivalent single current source ESCS or an equivalent single magnetic source ESMS at the reference source position, the ESCS or ESMS representing an electric heart vector EHV or a magnetic heart vector MHV, in relation to the reference direction, and
e. a means for registering the EHV and/or MHV during at least part of the heart activity in a vector magnetocardiogram.
